# EUROPÄISCHE PATENTSCHRIFT

(11) **EP 3 905 975 B1**
(45) Veröffentlichungstag und Bekanntmachung des Hinweises auf die Patenterteilung: **30.08.2023**
(21) Anmeldenummer: 20700331.0
(22) Anmeldetag: 02.01.2020
(51) Int. Cl.: A61B 17/88

(54) **FÜGEVERFAHREN FÜR EINE MEDIZINTECHNISCHE VORRICHTUNG**
JOINING METHOD FOR A MEDICAL DEVICE
PROCÉDÉ D'ASSEMBLAGE D'UN DISPOSITIF MÉDICAL

(30) Priorität: 02.01.2019 DE 102019100016
(43) Veröffentlichungstag der Anmeldung: 10.11.2021
(73) Patentinhaber: Aesculap AG, 78532 Tuttlingen (DE)
(72) Erfinder: ACKERMANN, Janina, 78532 Tuttlingen (DE); BARTHELMES, Sven, 78576 Emmingen-Liptingen (DE); DAHMEN, Jan, 78606 Seitingen-Oberflacht (DE); DEUTSCHENDORF, Andreas, 78549 Spaichingen (DE); MATTES, Till, 78579 Neuhausen ob Eck (DE); PLEIL, Elke, 78073 Bad Dürrheim (DE)
(74) Vertreter: Winter, Brandl - Partnerschaft mbB
(86) Internationale Anmeldenummer: PCT/EP2020/050037
(87) Internationale Veröffentlichungsnummer: WO 2020/141197

(56) Entgegenhaltungen:
- EP-A1- 2 669 032
- WO-A1-01/80768
- WO-A1-2004/052244
- US-A1- 2004 117 024
- US-A1- 2012 130 502

## Beschreibung

### Technischer Hintergrund

Medizintechnische Vorrichtungen unterliegen hohen Anforderungen hinsichtlich Funktionssicherheit, Toleranzen und insbesondere Sterilität / Sterilerhaltung. Dies gilt insbesondere für Verbindungsstellen solcher Vorrichtungen, an welchen zwei Bauteile fest miteinander verbunden werden. Nachfolgend wird beispielhaft auf die Verbindungsstelle eines chirurgischen Instruments mit einem Griff und einem Arbeitsende, beispielsweise einem chirurgischen Hammer Bezug genommen. Es ist jedoch anzumerken, dass die gleiche Problematik auf eine Vielzahl weiterer medizintechnischer Vorrichtungen und verschiedener Verbindungsstellen derselben anwendbar ist.

### Stand der Technik

Nach derzeitigem Stand der Technik werden Griffe und Arbeitsenden (Effektoren) gefügt, indem diese beispielsweise händisch miteinander verpresst und gegen axiales und radiales Verrutschen durch einen Querstift oder Sicherungsstift gesichert werden. Der Querstift wird dabei in eine Bohrung eingepresst, welche durch den Griff und das Arbeitsende verläuft, und muss anschließend durch Schleifen eingepasst und die Oberfläche abgebürstet werden. Diese Arbeitsgänge sind kosten- und zeitintensiv. Ferner entstehen bzw. bleiben bei solchen Pressverbindungen Spalte, in welchen sich Ablagerungen bilden und Blut, Keime, Schmutz sowie chemische Aufbereitungsmittel etc. hineindiffundieren, welche bei einer Reinigung nicht herausgelöst werden können. Dies führt nachteiliger Weise zu Kontamination und Korrosion der Verbindungsstelle. Um dies zu vermeiden wird ggf. die Verbindungsstelle nachträglich durch Schweißen oder Kleben abgeschirmt, was das Fertigungsverfahren zusätzlich verkompliziert und verteuert. Andersartige Fügeverfahren, wonach unter thermischer Belastung/Einwirkung zwei Bauteile einer medizinischen Vorrichtung miteinander verbunden werden, wurden in der Vergangenheit im Wesentlichen vernachlässigt, da grundsätzlich mit Wärmeverzug zumindest eines der zu fügenden Bauteile außerhalb der vorgeschriebenen Toleranzen gerechnet wurde.

Stand der Technik zu Fügeverfahren im Bereich der Medizintechnik ist beispielsweise bekannt aus US 2012/130502 A1, welches ein Verfahren und Vorrichtungen für den orthopädischen Ersatz der Hüfte durch ein modulares Prothesensystem offenbart, wobei Strukturen und Techniken zur Fixierung oder Verbesserung der Verbindung von Implantatkomponenten untereinander bereitgestellt sind, beispielsweise durch Erhöhung der Verbindung in einer Presspassung mit Kühlung der Komponenten vor dem Zusammenbau.

Aus US 2004/117024 A1 sind modulare orthopädische Implantate bekannt, die aus Basis-, Körper- und Schaftkomponenten bestehen. Die Basis und der Schaft sind verbindbar. Verbindungen können Kombinationen von Presspassungen wie Presspassungen, Mehrfachpresspassungen und konische Passungen umfassen. Auch Schrumpfverbindungen werden erörtert, bei denen zunächst die weibliche Komponente auf eine Temperatur oberhalb ihrer Arbeitstemperatur erwärmt und dann die männlichen und weiblichen Komponenten zusammengefügt werden, um sie abkühlen zu lassen.

Weitere medizinische Implantate, bei welchen Komponenten über eine Schrumpfverbindung verbunden werden, sind z.B. aus WO 2004/052244 A1 und WO 01/80768 A1 bekannt.

### Zusammenfassung der Erfindung

Der Erfindung liegt eine Aufgabe zugrunde, Nachteile des Stands der Technik zu reduzieren oder zu vermeiden sowie ein kostengünstiges Fügeverfahren für eine stabile, unverlierbare Verbindung zweier Bauteile einer medizintechnischen Vorrichtung bereitzustellen, welche den hohen Anforderungen an Sterilität genügt.

Diese Aufgabe wird durch ein Fügeverfahren gemäß Anspruch 1 und die Verwendung eines entsprechenden Fügeverfahrens zur Herstellung einer medizintechnischen Vorrichtung (z.B. medizinisches/chirurgisches Instrument) gemäß Anspruch 13 gelöst.

Vorteilhafte Ausgestaltungen der Erfindung sind Gegenstand der Unteransprüche.

Der Kern der vorliegenden Erfindung besteht demzufolge darin, zwei miteinander zu fügende Bauteile einer medizinischen Vorrichtung, insbesondere eines medizinischen (chirurgischen) Instruments durch thermisches Aufschrumpfen zu verbinden. Dabei ist erfindungsgemäß darauf zu achten, dass vorzugsweise nur jenes Bauteil im Wesentlichen thermisch bearbeitet wird, an das gegenüber dem anderen Bauteil geringere Anforderungen beispielsweise hinsichtlich Maßtoleranzen und/oder Materialeigenschaften gestellt sind. Alternativ oder zusätzlich sollte bevorzugt nur jenes Bauteil im Wesentlichen thermisch bearbeitet werden, das gegenüber dem anderen Bauteil eine größere Wärmekapazität (z.B. infolge größere Masse) aufweist. Alternativ oder zusätzlich sollte bevorzugt nur jenes Bauteil im Wesentlichen thermisch bearbeitet werden, das aufgrund seiner Struktur und/oder seiner räumlichen Abmessungen/Form gegenüber dem anderen Bauteil geringere Verzugstendenzen infolge Wärmeeintrag zeigt. Alternativ oder zusätzlich sollte nur jenes Bauteil im Wesentlichen thermisch bearbeitet werden, welches aus einen Material besteht, das gegenüber dem Material des anderen Bauteils thermische Vorteile hat, beispielsweise sich bei einer gegenüber dem anderen Bauteil niedrigeren Temperatur ausreichend deformiert, um einen Aufschrumpfprozess zu ermöglichen.

Beispielsweise kann eine medizintechnische Vorrichtung (medizinisches Instrument) u.a. aus einem (langgestreckten/schlanken) Instrumentenschaft und einem damit verbundenen/zu verbindenden Instrumentengriff und/oder einem damit verbundenen/zu verbindenden Effektor (z.B. Hammerkopf, Kralle, etc.) bestehen. In einen solchen Beispiel würde erfindungsgemäß der gegenüber dem Instrumentenschaft robuster aufgebaute Effektor (kürzer, dicker, massiver, etc.) jenes Bauteil darstellen, welches gegenüber dem Instrumentenschaft eine geringere thermisch verursachte Verzugstendenz aufzeigt und damit einer thermischen Behandlung für ein nachfolgendes Aufschrumpfen (ausschließlich) unterzogen wird.

Die der Erfindung zugrunde liegende Aufgabe wird im Konkreten durch ein Fügeverfahren für eine medizintechnische Vorrichtung, insbesondere ein chirurgisches Instrument oder Implantat, mit einem beispielsweise aus Titan gefertigten Schaft und einem darauf zu befestigenden, beispielsweise aus Stahl gefertigten Aufsatz (Griff, Effektor, etc.) gelöst, wobei das Fügeverfahren folgende Schritte aufweist:
- Zunächst wird der Aufsatz aufgeheizt oder wird der Schaft abgekühlt, sodass dieser sich aufgrund der Wärmeausdehnung bzw. -schrumprung gemäß dem materialspezifischen Ausdehnungskoeffizienten ausdehnt bzw. schrumpft, wodurch der Innendurchmesser einer (Aufnahme-)Öffnung des Aufsatzes größer als ein Außendurchmesser des (einzusetzenden) Schafts ist/wird.
- Anschließend wird der Schaft in die Öffnung des Aufsatzes eingesetzt. D. h., aufgrund der Wärmeausdehnung des Aufsatzes bzw. der Wärmeschrumpfung des Schafts ist/wird die Öffnung gegenüber dem Schaftquerschnitt vergrößert und sind der Schaft und der Aufsatz in diesem Zustand in Schaft-Axialrichtung zueinander frei beweglich.
- Anschließend, d.h. nach Einsetzen des Schafts in die entsprechende Aufsatzöffnung wird der Aufsatz (aktiv oder passiv) abgekühlt, um diesen im Wesentlichen spaltfrei auf den Schaft aufzuschrumpfen bzw. wird der Schaft (aktiv oder passiv) erwärmt, um diesen innerhalb des Aufsatzes auszudehnen, sodass der Schaft und die Öffnung eine Übermaß- oder Presspassung (also eine Pressverbindung) ausbilden, vorzugsweise bei der im Betrieb maximal zu erwarteten Umgebungstemperatur, weiter vorzugsweise bei Raumtemperatur.

Dieses bei medizinischen Vorrichtungen erstmalig angewandte Fügeverfahren ist zumindest teil-, ggf. auch vollautomatisierbar und erfordert keine oder nur wenige, wenig anspruchsvolle, händisch durchzuführende Schritte (einen Arbeitsgang) seitens eines Anwenders. D. h., Handarbeit wird reduziert und es entfallen bei der Montage einige kostenintensive, manuelle Arbeitsgänge sowie der Querstift. Es wird also eine einfache, schnelle und kostengünstige sowie validierbare und unverlierbare Verbindung zweier Bauteile der medizinischen Vorrichtung ermöglicht, welche auch den strengen Anforderungen an Sterilität/Reinigbarkeit genügt. Darüber hinaus werden auch das äußere Erscheinungsbild, die Qualität und die Belastbarkeit der Verbindung und der Vorrichtung verbessert, sodass höhere (Halte- und/oder Torsions-) Kräfte als bei bisherigen Lösungen übertragbar sind. Zudem werden eine Wiederholungsgenauigkeit / Reproduzierbarkeit und Prozesssicherheit des Fügeverfahrens verbessert. Demgemäß ist der Prozess dazu geeignet, als Basis für eine Vereinheitlichung von diversen medizintechnischen Verbindungsstellen oder Schnittstellen zu dienen und dadurch die Kosten der Gesamtproduktion zu senken. Besonders wichtig ist zudem, dass durch dieses Verfahren aufgrund einer hohen Anpresskraft innerhalb der Übermaßpassung gewährleistet ist, dass ein Spalt zwischen dem Schaft und dem Aufsatz während des Abkühlens im Wesentlichen vollständig geschlossen wird und nachweislich keine Kontamination und Korrosion innerhalb der Verbindungsstelle beim beabsichtigten Betreib der medizinischen Vorrichtung stattfindet, insbesondere bei einer Verwendung im chirurgischen Umfeld und einer anschließenden chemischen/thermischen (Sterilisations-)Aufbereitung.

Der Schaft kann einen hinterschneidungsfreien, runden oder polygonalen, beispielsweise vier- oder sechseckigen Querschnitt haben. Ferner kann der Aufsatz an einer Seite, an welcher der Schaft aus dem darauf befestigten Aufsatz hervorragt (im Falle eines chirurgischen Instruments einem proximalen Ende), schräg zulaufend sein, sodass an einer Übergangsstelle zwischen dem Schaft und dem Aufsatz ein Winkel größer 90° ausgebildet ist, wodurch die Übergangsstelle für eine Reinigung und Sterilisierung besser zugänglich ist und/oder an diese Übergangsstelle weniger anfällig für die Bildung von Ablagerungen ist. Darüber hinaus ist zu beachten, dass der Schaft und der Aufsatz aus unterschiedlichen Materialien gefertigt sein können. Bei dem vorstehenden Beispiel, gemäß welchem der Schaft aus Titan und der Aufsatz aus Stahl hergestellt ist, können ggf. beide Bauteile gemeinsam aufgeheizt werden, da Stahl eine höhere Wärmeausdehnung bzw. einen höheren Längenausdehnungskoeffizienten aufweist und somit die notwendige Aufweitung der Öffnung des Aufsatzes auch bei dem gemeinsamen Aufheizen erreichbar ist, ohne dass sich beim (Titan-)Schaft übergebührliche Verzugstendenzen zeigen.

Es ist anzumerken, dass aufgrund der speziellen Anforderungen und Materialien medizintechnischer Produkte insbesondere die geometrische Auslegung einer solchen Verbindungsstelle und die Auswahl des verwendbaren Temperaturbereichs wichtige Aspekte darstellen, auf welche nachfolgend genauer eingegangen wird.

Nach einem Aspekt sind für eine Steuerung, bevorzugt eine Regelung, zum Steuern oder Regeln des Fügeverfahrens Parameter frei programmierbar oder einstellbar, um eine Anpassung des Fügeverfahrens an abweichende Umgebungsbedingungen oder zum Fügen unterschiedlicher Vorrichtungen mit unterschiedlichen Abmessungen und/oder Materialien zu ermöglichen. Einstellbare Parameter sind hierbei beispielsweise die Heiz- oder Kühlleistung, die Aufheiz- oder Abkühlzeit und der Heiz- oder Kühlleistungsverlauf. Somit kann das erfindungsgemäße Verfahren an einer einzelnen Maschine oder Station zum Durchführen dieses Verfahrens für die Fertigung unterschiedlicher medizintechnischer Vorrichtungen angewendet werden.

Erfindungsgemäß findet während des Fügeverfahrens ferner ein Überwachen der Temperatur des Aufsatzes und/oder des Schafts sowie weiter bevorzugt der Aufheizund/oder Abkühlzeit statt. Hierfür kann beispielsweise zumindest ein Pyrometer als ein Temperatursensor eingesetzt werden. Dadurch kann gewährleistet werden, dass der Schaft zum richtigen Zeitpunkt leicht in die Öffnung eingesetzt werden kann und dass ein Beenden des Fügeverfahrens nicht zu früh erfolgt, wodurch ggf. der Aufsatz und der Schaft verrutschen oder sich sogar voneinander lösen könnten. Zudem kann verhindert werden, dass der Aufsatz oder ggf. auch der Schaft bei Kontakt mit dem Aufsatz zu stark erwärmt bzw. abgekühlt werden, um bei oder nach der Erwärmung oder dem Abkühlen keine Nachteile hinsichtlich der mechanischen Eigenschaften der entsprechenden Bauteile zu generieren. Das Pyrometer kann unmittelbar vor Beginn der Messung und/oder während der Messung händisch oder automatisiert z.B. mit Hilfe von Markern korrekt ausgerichtet werden. Alternativ können statt eines oder mehrerer Pyrometer beispielsweise auch Kontaktthermometer o. Ä. angebracht werden.

Insbesondere ist es von Vorteil, wenn die Temperatur des Aufsatzes bzw. des Schafts an zumindest zwei Punkten (Positionen) überwacht wird: nahe, vorzugsweise unmittelbar an der Öffnung (d. h. an deren Innenumfangsfläche oder unmittelbar neben oder an einer Kante an einem Öffnungsausgang) bzw. dem Außenumfang des einen in den Aufsatz einzusetzenden Ende des Schafts sowie an einem dazu beabstandeten Aufsatzrandbereich (d.h. möglichst weit entfernt von der Öffnung) bzw. an einem dazu beabstandeten Schaftbereich. Dies ermöglicht es, bei der Einstellung von Prozessparametern die Temperaturen an unterschiedlichen relevanten Punkten zu überwachen, zu vergleichen und somit auch die Wärmeleitung innerhalb des Aufsatzes bzw. des Schafts während des Aufheizens und ggf. des Abkühlens zu überwachen, um einen optimalen Prozessverlauf einzustellen oder zu regeln.

Eine Temperaturmessung am Schaft (z.B. an einem ortsfesten oder während des Fügeverfahrens ortsveränderlichen Punkt) ist beispielsweise von Vorteil, wenn man diesen herunterkühlt. Alternativ oder zusätzlich ist dies von Vorteil, um eine geforderte Mindesttemperatur des Aufsatzes im Verhältnis zu der Schaft- oder Raumtemperatur, welche einander ggf. entsprechen (insbesondere wenn der Schaft nicht geheizt oder gekühlt wird), und/oder einen Mindesttemperaturunterschied zwischen dem Aufsatz und/oder dem Schaft und/oder der Raumtemperatur zu bestimmen oder vorzugeben. Darüber hinaus ist es vorteilhaft, wenn im axialen Verlauf der Öffnung und/oder des Schafts, insbesondere der Schaftspitze (eines Schaftabschnitts, welcher in der Öffnung aufgenommen wird), eine Temperaturüberwachung durchgeführt wird, um nach dem Abkühlen axiale Spannungen zu vermeiden oder zu reduzieren. In anderen Worten ausgedrückt, wird bevorzugt axial entlang des Schafts bzw. der Schaftspitze (entlang dessen/deren axialer Erstreckung / Axialverlauf) und/oder axial entlang einer Öffnungsinnenumfangsfläche (entlang des Axialverlaufs innerhalb der Öffnung) (ortsaufgelöster und/oder zeitaufgelöster) Temperaturverlauf bestimmt, wofür beispielsweise verteilt in Axialrichtung am Schaft und/oder innerhalb der Öffnung zwei oder mehr Punkte zur Temperaturmessung bzw. Messpunkte vorgesehen sein können.

Bevorzugt wird zum Aufheizen des Aufsatzes Induktionswärme eingesetzt oder für ein Abkühlen des Schafts Stickstoff eingesetzt. Insbesondere ein Aufheizen mittels Induktionswärme ist eine einfache, sichere, schnell reagierende und gut steuerbare Heizmethode, bei welcher offene Wärmequellen vermieden und dadurch eine Verletzungsgefahr reduziert wird. Alternativ kann auch eine Heizplatte bzw. eine Widerstandsheizung o.Ä. verwendet werden.

Nach einem Aspekt der Erfindung kann im Rahmen des Fügeverfahrens ferner ein Einstellen eines Wärmeeintrags beim Aufheizen abhängig von Bauteil-Parametern des Aufsatzes und ggf. des Schafts erfolgen. Beispielsweise können die Geometrie, die Oberflächenbeschaffenheit, die Dimensionierung, der Wärmebehandlungszustand, die Ausdehnungskoeffizienten und/oder die Korrosionsbeständigkeit des Aufsatzes und/oder des Schafts als Bauteil-Parameter berücksichtigt werden. Insbesondere ist die Berücksichtigung des Ausdehnungskoeffizienten und des Wärmebehandlungszustands, also von Eigenschaften von speziell in der Medizintechnik verwendeten Materialien, wichtig, um die Korrosionsbeständigkeit nicht negativ zu beeinflussen.

Die Übermaßpassung des Schafts und des Aufsatzes ist bevorzugt derart dimensioniert, dass der Schaft und der Aufsatz im Betrieb axial und radial unlösbar (unverlierbar) miteinander verbunden sind. Dies ist wichtig, um die Funktionssicherheit der Vorrichtung zu gewährleisten. Dabei ist berücksichtigt, dass der Schaft und der Aufsatz ggf. aus unterschiedlichen Materialien mit unterschiedlichen Wärmedehnungskoeffizienten gefertigt sind und die Übermaßpassung derart ausgelegt ist, dass die Pressverbindung zumindest in einem für den Betrieb der Vorrichtung vorgesehenen Temperaturbereich sichergestellt ist. Weiter bevorzugt ist die entsprechende Verbindung durch erneutes Aufheizen reversibel lösbar, um beispielsweise in einem Servicefall eine schnelle und einfache Reparatur oder Austausch des Schafts und/oder des Aufsatzes zu ermöglichen. Es hat sich als sinnvoll erwiesen, dass die Übermaßpassung ein Übermaß zwischen 20 und 55 µm aufweist. Je nach Anwendungsfall der medizintechnischen Vorrichtung können dabei unterschiedliche Anforderungen an die Festigkeit der Verbindung und kann somit das Übermaß unterschiedlich ausgelegt werden. Beispielsweise kann in einer ersten, niedrigbelasteten Anwendung ein kleineres Übermaß, beispielsweise von 20 bis 25 µm, in einer zweiten mittleren Anwendung ein mittleres Übermaß, beispielsweise von 35 bis 40 µm, oder in einer dritten, hochbelasteten Anwendung ein großes Übermaß, beispielsweise von 50 bis 55 µm, vorgesehen werden. D. h., die geometrische Auslegung des Schafts und des Aufsatzes, insbesondere der Öffnung des Aufsatzes, ist von einem Anwendungsfall des Produkts abhängig.

Bevorzugt liegt eine Aufheiztemperatur des Aufsatzes zwischen 50 und 350°C, vorzugsweise zwischen 300 und 350°C. Dies ermöglicht eine ausreichende Aufweitung der Öffnung, ohne nachteilige Auswirkungen auf die mechanischen Eigenschaften des Schafts oder des Aufsatzes zu haben.

Nach einem weiteren Aspekt Erfindung kann das Einsetzen des Schafts in die Öffnung des Aufsatzes über eine Lineareinheit erfolgen. Dies ermöglicht es, neben dem Aufheizen und dem Abkühlen auch das Einsetzen des Schafts in die Öffnung des Aufsatzes zu automatisieren oder zumindest zu vereinfachen und somit die Fertigungskosten weiter zu senken. Ferner ist dadurch eine Einsetztiefe des Schafts genau steuerbar und reproduzierbar und wird eine Handhabung durch den Anwender weiter reduziert, wodurch sowohl die Verletzungsgefahr für den Anwender an dem heißen Aufsatz als auch menschliche Fehler reduziert werden. Die Lineareinheit kann den Aufsatz bereits an der Heizung bzw. der Kühleinheit übernehmen oder diesen schon während des Heizens bzw. des Kühlens halten. Wenn der Aufsatz nach dem Aufheizen bzw. Kühlen von der Heizung bzw. Kühleinheit entfernt wird bzw. zum Aufsetzen auf den Schaft bewegt wird, steht die Heizung bzw. Kühleinheit bereits zum Aufheizen des nächsten Aufsatzes bereit und kann eine Taktung der Fertigung erhöht werden. In diesem Fall ist die Lineareinheit von besonderem Vorteil, da das Einsetzen des Schafts in den Aufsatz angesichts des teilweise hohen Miniaturisierungsgrads der medizintechnischen Vorrichtungen ggf. sehr genau und sehr schnell erfolgen muss, da die Abmessungen und Toleranzen sehr klein sind und die Bauteile schnell abkühlen bzw. sich schnell erwärmen. Würde dies händisch durch einen Anwender vorgenommen, wäre ein Fehlerpotential voraussichtlich hoch. Alternativ kann durch die Lineareinheit der Schaft gehalten und in die Öffnung des positionsfesten Aufsatzes eingesetzt/eingeführt werden. In diesem Fall muss der Aufsatz bzw. der Schaft im aufgeheizten bzw. abgekühlten Zustand gar nicht bewegt werden und reicht ein Ausschalten der Heizung bzw. der Kühleinheit, um ein Abkühlen bzw. Erwärmen des Aufsatzes bzw. des Schafts einzuleiten.

Eine medizintechnische Vorrichtung, insbesondere ein chirurgisches Instrument oder Implantat, gemäß der vorliegenden Offenbarung hat einen Aufsatz mit einer Öffnung und eine in die Öffnung eingesetzten Schaft, wobei ein Außendurchmesser des Schafts und ein Innendurchmesser der Öffnung des (abgekühlten) Aufsatzes dimensioniert sind, um eine Übermaßpassung bzw. Pressverbindung auszubilden, und der Aufsatz durch das vorstehend beschriebene Fügeverfahren mit dem Schaft verbunden ist. Vorzugsweise ist der Schaft ein Stiel oder Handgriff und ist der Aufsatz ein Arbeitsende oder Werkzeugkopf eines medizinischen Instruments. Hinsichtlich Vorteilen und weiteren Details dieser Vorrichtung wird auf die vorstehende Beschreibung des zugehörigen Fügeverfahrens verwiesen.

Darüber hinaus wird die Aufgabe gelöst, durch eine Verwendung des vorstehend beschriebenen Fügeverfahrens zur Herstellung einer medizinischen Vorrichtung, insbesondere eines medizinischen Instruments oder Implantats, bevorzugt gemäß der vorstehenden Beschreibung, welche einen Aufsatz und einen in eine Öffnung des Aufsatzes eingesetzten Schaft aufweist. Hinsichtlich Vorteilen und Ausgestaltungen des Fügeverfahrens sowie der medizinischen Vorrichtung wird auf die vorstehende Beschreibung verwiesen.

### Figurenbeschreibung

Fig. 1 ist eine schematische Darstellung eines erfindungsgemäßen Fügeverfahrens nach einer Ausführungsform, wobei Fig. 1A - C aufeinanderfolgende Schritte des Fügeverfahrens zeigen.
Fig. 2 zeigt eine beispielhafte Fertigungsanordnung für das Fügeverfahren.
Fig. 3 zeigt ein Beispiel für eine andere Ausführungsform des Aufsatzes.

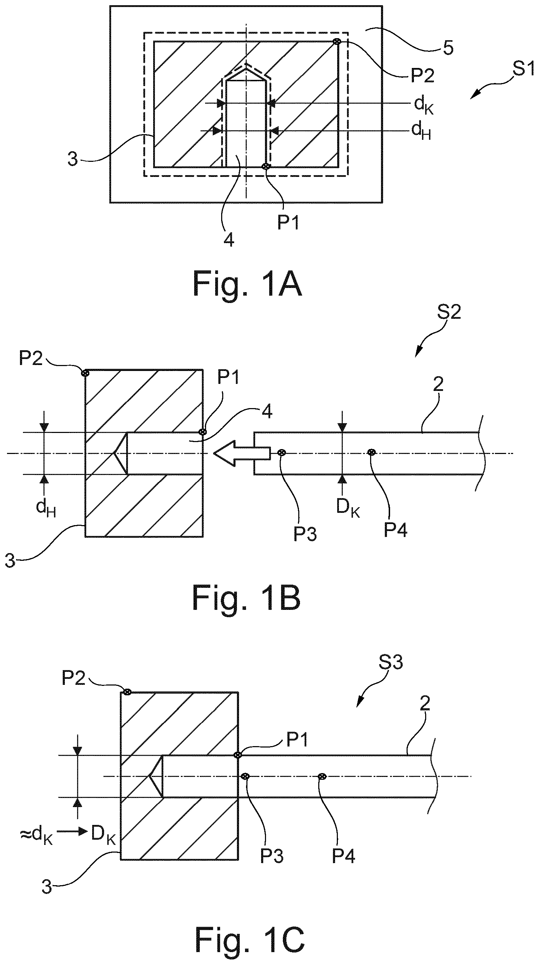

Fig. 1 zeigt eine Ausführungsform eines erfindungsgemäßen Fügeverfahrens für eine medizintechnische Vorrichtung 1, welche zur Veranschaulichung hier als chirurgisches Instrument mit einem Schaft oder Stiel 2 und einem einfachen, zylinderoder quaderförmigen Aufsatz oder Arbeitsende 3, z. B. nach Art eines chirurgischen Hammers, dargestellt ist. Je nach Anwendung sind jedoch beliebige anders geformte Arbeitsenden 3 auswählbar.

Fig. 1A veranschaulicht den Schritt des Aufheizen S1. Ein Arbeitsende 3, welches eine Öffnung 4, in diesem Beispiel eine Bohrung oder ein Sackloch, aufweist, ist an einer Induktionsheizung 5 angeordnet und wird durch Induktionswärme erhitzt. Dabei wird vorzugsweise eine Temperatur (ggf. ein zeitlicher und/oder örtlicher (axialer) Temperaturverlauf) des Aufsatzes oder des Arbeitsendes 3 an einem Punkt P1 nahe eines Rands der Öffnung und/oder an einem davon beabstandeten Punkt P2 des Aufsatzes gemessen. Alternativ oder zusätzlich wird optional eine Temperatur des Schafts oder Stiels 2 an einem Punkt P3 nahe eines in die Öffnung 4 einzusetzenden Stiel-/Schaftende sowie einem Punkt P4 an einem weiteren dazu beabstandeten Stiel/Schaftbereich gemessen. Aufgrund der Wärmeausdehnung eines Materials des Arbeitsendes 3 dehnt sich das Arbeitsende 3 aus, wodurch auch die Öffnung 4 vergrößert wird. D. h., die Öffnung 4 hat bei Raumtemperatur einen ersten "kalten" Innendurchmesser d_{K} und hat im aufgeheizten Zustand einen zweiten, "heißen" Innendurchmesser d_{H}, welcher größer als der kalte Innendurchmesser d_{K} ist.

Nachdem das Arbeitsende 3 auf eine bestimmte Temperatur und/oder während einer bestimmten Aufheizzeit aufgeheizt wurde (wobei die Temperatur z.B. an dem Punkt P1 und/oder P2 gemessen werden kann), wird der Stiel 2, dessen "kalter" Außendurchmesser D (d. h., vorzugsweise bei Raum- oder maximale im Betrieb zu erwartender Umgebungstemperatur, wobei die Stiel- oder Schafttemperatur z.B. an dem Punkt P3 und/oder P4 an dem Stiel 2 messbar/überwachbar ist) bezüglich des kalten Innendurchmessers d_{K} des Arbeitsende 3 ein bestimmtes Übermaß hat, jedoch kleiner als der zu diesem Zeitpunkt vorliegende heiße Innendurchmesser d_{H} ist, in Längsrichtung des Stiels 2 in die Öffnung 4 eingesetzt (S2).

Anschließend kühlt das Arbeitsende 3 ab (S3), wodurch auch die Öffnung 4 schrumpft/schmaler wird und das Arbeitsende 3 auf dem Stiel 2 aufschrumpft. D. h., wie in Fig. 1C gezeigt, stellt sich aufgrund einer Übermaßpassung/Presspassung eine Pressverbindung des Stiels 2 mit dem Arbeitsende 3 ein. Vorzugsweise wird währenddessen weiterhin die Temperatur des Schafts/Stiels 2 und/oder des Arbeitsendes 3 entsprechend an den Punkten P1 und/oder P2 und/oder P3 und/oder P4 gemessen. Eine Innenumfangsfläche der Öffnung 4 ist dabei aufgrund des Übermaßes des Stiels 2 bei Raumtemperatur axial und radial unverschiebbar (gegenüber bei einem vorgesehenen Einsatzzweck auftretenden Kräften) mit eine Außenumfangsfläche des Stiels 2 verpresst.

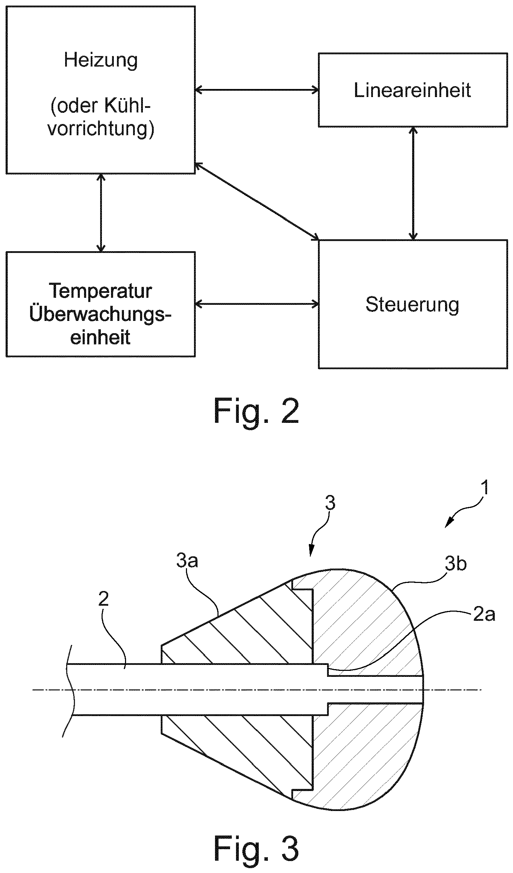

Fig. 2 zeigt eine beispielhafte Fertigungsanordnung für das vorstehend beschriebene Fügeverfahren. Eine Heizung 5, in diesem Beispiel eine Induktionsspule, ist bereitgestellt, welche in Schritt S1 zum Aufheizen des Arbeitsendes 3 dient. Dabei erfasst eine Temperaturüberwachungseinheit 6 beispielsweise über ein Pyrometer eine Temperatur des Arbeitsendes 3 an mehreren Stellen (Schritt S4) und ermittelt daraus eine Temperaturabweichung sowie ggf. einen Wärmeeintrag. Eine Steuerungseinheit 7, welche die Aufheizzeit überwacht sowie eine Soll-Temperatur und ggf. einen Temperaturverlauf oder Wärmeeintrag vorgibt, empfängt Überwachungssignale der Temperaturüberwachungseinheit, und steuert auf deren Basis die Heizung 5 (Schritt S5).

Ist eine Soll-Temperatur erreicht, wird ein Signal, z.B. ein Ton- oder Lichtsignal, ausgegeben und das Arbeitsende 3 durch einen Anwender an eine Lineareinheit 8 übergeben oder wird das Arbeitsende 3 vollautomatisch durch die Lineareinheit 8 übernommen. Die Lineareinheit 8 dient dazu, das Arbeitsende 3 in Schritt S2 sicher und gleichmäßig auf den Stiel 2 aufzuschieben/aufzusetzen, sodass dieser nach dem Abkühlen (S3) des Arbeitsendes 3 korrekt darin positioniert ist. Alternativ kann auch das Aufsetzen händisch durch den Anwender erfolgen. Erfolgt das Aufsetzen oder die Übergabe an die Lineareinheit 8 durch den Anwender, kann die Steuerungseinheit 7 zudem konfiguriert sein, um die Heizung 5 derart anzusteuern, dass das Arbeitsende 3 während einer bestimmten Zeit auf der Soll-Temperatur gehalten wird. Anschließend kann die Steuerungseinheit 7 während des Abkühlens die Abkühlzeit sowie über die Temperaturüberwachungseinheit 6 die Temperatur des Arbeitsendes 3 überwachen, um zu bestimmen, wann das Arbeitsende 3 und der Stiel 2 ausreichend fest aufeinander sitzen und ggf. ohne Verletzungsgefahr für einen Anwender gehandhabt werden können. Zu diesem Zeitpunkt kann ein weiteres Signal ausgegeben werden.

Fig. 3 zeigt ein Beispiel für eine andere Ausführungsform des Aufsatzes oder Arbeitsendes 3. Darin sind mehrere voneinander unabhängig zu betrachtende Aspekte dargestellt, welche in beliebigen Kombinationen oder einzeln bei einem erfindungsgemäßen Arbeitsende 3 realisierbar sind. Ein erster Aspekt betrifft einen im Wesentlichen tropfenförmigen Querschnitt des Arbeitsendes 3, wobei die Spitze des Tropfens in Richtung des Schafts oder Stiels 2 weist. In anderen Worten läuft der Querschnitt des Arbeitsendes 3 in Richtung des Schafts oder Stiels 2 (wo dieser proximal aus dem Arbeitsende hervorsteht) zu. Dies verbessert die Zugänglichkeit der Kante an dem Übergang zwischen dem Arbeitsende 3 und dem Stiel 2 zu Reinigungsund/oder Sterilisationszwecken.

Ein zweiter Aspekt betrifft ein Arbeitsende 3, welches mehrteilig ist. Dabei ist ein erstes, proximales (also näher an einem zu handhabenden proximalen Stielende liegendes) Aufsatzteil 3a auf den Stiel 2 aufgeschrumpft bzw. aufschrumpfbar und ein zweites, distales Aufsatzteil 3b kann beispielsweise als ein Einwegteil ausgebildet und auf das Instrument (d. h. auf das bereits mit dem Stiel 2 verbundene proximale Aufsatzteil 3a) aufsteckbar sein. Alternativ kann das distale Aufsatzteil 3b auch zuerst mit dem proximalen Aufsatzteil 3a verbunden und anschließend gemeinsam mit diesem auf dem Stiel 2 aufgeschrumpft werden, wobei die vorgesehenen Schnittstellen und Durchmesser exakt aufeinander abgestimmt werden müssen, um einander während der Wärmedehnung und dem anschließenden Schrumpfen nicht gegenseitig zu beschädigen. Das distale Aufsatzteil kann in Längsrichtung des Stiels 2 distal geschlossen oder offen sein.

Ein dritter Aspekt betrifft eine Festlegung der Position des Stiels 2 bezüglich des Arbeitsendes 3 in Längsrichtung des Stiels 2. Der Stiel 2 kann eine Stufe oder Schulter 2a ausbilden, welche dazu vorgesehen ist, in Längsrichtung in Anlage mit einer komplementären Schulter oder Stufe des Arbeitsendes 3 in Anlage zu kommen.

Ein vierter Aspekt betrifft eine Ausbildung der Öffnung 4 des Aufsatzes 3 als ein Durchgangsloch. Dadurch kann verhindert werden, dass in einer Tasche zwischen einer Öffnungswand und dem distalen Ende des Stiels 2 Luft eingeschlossen wird, welche die korrekte Positionierung in axialer Richtung beeinträchtigen könnte. In diesem Fall ist es vorteilhaft, wenn der Stiel 2 in Längsrichtung des Stiels 2 derart in dem Aufsatz 3 positioniert wird, dass ein distales Stielende bündig mit dem Aufsatz 3 abschließt, um eine zusätzliche Kante, welche zu reinigen wäre, zu vermeiden.

Es ist anzumerken, dass die vorstehend beschriebene Ausführungsform auch derart adaptiert werden kann, dass anstelle der Heizung auch eine Kühlvorrichtung vorgesehen werden kann und anstelle eines Aufheizens des Aufsatzes auch ein Abkühlen des Schafts durch diese Kühlvorrichtung vorgesehen werden kann, wobei der Schaft nach dem Einsetzen des Schafts in die Öffnung des Aufsatzes erwärmt wird, um die Presspassung herzustellen.

### Bezugszeichenliste

- 1: medizintechnische Vorrichtung
- 2: Schaft (Stiel)
- 2a: Stufe/Schulter
- 3: Aufsatz (Arbeitsende)
- 3a: proximales Aufsatzteil
- 3b: distales Aufsatzteil
- 4: Öffnung
- 5: (Induktions-) Heizung
- 6: Temperaturüberwachungseinheit
- 7: Steuerungseinheit
- 8: Lineareinheit
- P1: (Mess-) Punkt nahe der Öffnung
- P2: (Mess-) Punkt an einem zu dem Punkt P1 beabstandeten Aufsatzrandbereich
- P3: (Mess-) Punkt an einem Außenumfang eines in den Aufsatz einzusetzenden Ende des Schafts
- P4: (Mess-) Punkt an einem zu dem Punkt P3 beabstandeten Schaftbereich

## Patentansprüche

1. Fügeverfahren für eine medizintechnische Vorrichtung (1), welche ein chirurgisches Instrument oder Implantat mit einem Schaft (2) und einem darauf zu befestigenden Aufsatz (3) ist, wobei das Fügeverfahren die folgenden Schritte umfasst:
Aufheizen (S1) des Aufsatzes (3) oder Abkühlen des Schafts (2), sodass ein Innendurchmesser (d_{H}) einer Öffnung (4) des aufgeheizten Aufsatzes (3) größer als ein Außendurchmesser (D) des Schafts (2) ist,
anschließendes Einsetzen (S2) des Schafts (2) in die Öffnung (4) des Aufsatzes (3),
anschließendes Abkühlen (S3) des Aufsatzes (3), um diesen im Wesentlichen spaltfrei auf den Schaft (2) aufzuschrumpfen, bzw. Erwärmen des Schafts (2), um diesen innerhalb der Öffnung (4) des Aufsatzes (3) auszudehnen, sodass der Schaft (2) und die Öffnung (4) über eine Übermaßpassung verbunden sind, **dadurch gekennzeichnet, dass**
das Fügeverfahren weiterhin den folgenden Schritt umfasst:
Überwachen (S4) der Temperatur des Aufsatzes (3) bzw. des Schafts (2) an zumindest einem ersten Punkt (P1) nahe an der Öffnung (4) bzw. einem Außenumfang eines in den Aufsatz einzusetzenden Ende des Schafts (2) sowie einem zweiten Punkt an einem zu dem ersten Punkt (P2, P3) beabstandeten Aufsatzrandbereich bzw. Schaftbereich.

2. Fügeverfahren nach Anspruch 1, ferner mit dem Schritt Überwachen (S4) der Temperatur des Aufsatzes (3) und vorzugsweise einer Aufheiz- und/oder Abkühlzeit.

3. Fügeverfahren nach Anspruch 2, wobei die Temperatur des Aufsatzes (3) nahe, vorzugsweise unmittelbar, an der Öffnung (4), sowie an einem dazu beabstandeten Aufsatzrandbereich überwacht wird.

4. Fügeverfahren nach einem der vorstehenden Ansprüche, wobei zum Aufheizen (S1) des Aufsatzes (3) eine Induktionsheizung (5) eingesetzt wird.

5. Fügeverfahren nach einem der vorstehenden Ansprüche, ferner mit dem Schritt Einstellen (S5) eines Wärmeeintrags beim Aufheizen (S1) abhängig von Parametern des Schafts (2) und des Aufsatzes (3).

6. Fügeverfahren nach einem der vorstehenden Ansprüche, wobei die Übermaßpassung derart dimensioniert ist, dass der Schaft (2) und der Aufsatz (3) im Betrieb axial und radial unlösbar miteinander verbunden sind und vorzugsweise durch erneutes Aufheizen reversibel lösbar ist.

7. Fügeverfahren nach einem der vorstehenden Ansprüche, wobei die Übermaßpassung ein Übermaß zwischen 20 und 55 µm aufweist.

8. Fügeverfahren nach einem der vorstehenden Ansprüche, wobei der Schaft (2) aus Titan gefertigt ist und der Aufsatz (3) aus Stahl gefertigt ist.

9. Fügeverfahren nach einem der vorstehenden Ansprüche, **dadurch gekennzeichnet, dass** eine maximale Aufheiztemperatur des Aufsatzes (3) zwischen 50 und 350°C, vorzugsweise zwischen 300 und 350°C, liegt.

10. Fügeverfahren nach einem der vorstehenden Ansprüche, wobei das Einsetzen (S2) des Schafts (2) in die Öffnung (4) des Aufsatzes (3) über eine Lineareinheit (8) erfolgt.

11. Fügeverfahren nach einem der Ansprüche 1 bis 10, wobei der Schaft (2) ein Stiel oder Handgriff ist und/oder der Aufsatz (3) ein Arbeitsende oder Werkzeugkopf ist.

12. Fügeverfahren nach einem der Ansprüche 1 bis 11, wobei der Schaft (2) aus Titan gefertigt ist und/oder der Aufsatz (3) aus Stahl gefertigt ist.

13. Verwendung eines Fügeverfahrens nach einem der Ansprüche 1 bis 12 zur Herstellung einer medizinischen Vorrichtung (1), insbesondere eines medizinischen Instruments oder Implantats, welche einen Aufsatz (3) und einen in eine Öffnung (4) des Aufsatzes (3) eingesetzten Schaft (2) aufweist.

## Claims

1. A joining method for a medical device (1) which is a surgical instrument or implant having a shaft (2) and an attachment (3) to be fixed thereto, wherein the joining method comprises the following steps:
heating (S1) the attachment (3) or cooling of the shaft (2) so that an inner diameter (d_{H}) of an opening (4) of the heated attachment (3) is larger than an outer diameter (D) of the shaft (2),
subsequently inserting (S2) the shaft (2) into the opening (4) of the attachment (3),
subsequently cooling (S3) the attachment (3) in order to shrink it onto the shaft (2) substantially without a gap, or heating the shaft (2) in order to expand it within the opening (4) of the attachment (3), so that the shaft (2) and the opening (4) are connected via an interference fit, **characterized in that**
the joining method further comprises the following steps:
monitoring (S4) the temperature of the attachment (3) or respectively of the shaft (2) at at least a first point (P1) close to the opening (4) or respectively at an outer circumference of an end of the shaft (2) to be inserted into the attachment as well as at a second point at an attachment border region or respectively shaft region spaced apart from the first point (P2, P3).

2. The joining method according to claim 1, further comprising the step of monitoring (S4) the temperature of the attachment (3) and preferably a heating and/or cooling time.

3. The joining method according to claim 2, wherein the temperature of the attachment (3) is monitored closely, preferably directly, on the opening (4) as well as at an attachment border region spaced apart therefrom.

4. The joining method according to one of the preceding claims, wherein an induction heating (5) is used for heating (S1) the attachment (3).

5. The joining method according to one of the preceding claims, further comprising the step of adjusting (S5) a heat input during heating (S1) depending on parameters of the shaft (2) and of the attachment (3).

6. The joining method according to one of the preceding claims, wherein the interference fit is dimensioned in such a way that the shaft (2) and the attachment (3) are axially and radially connected to each other in an inseparable manner during operation and can preferably be reversibly separated by reheating.

7. The joining method according to one of the preceding claims, wherein the interference fit has an allowance for interference between 20 and 55 µm.

8. The joining method according to one of the preceding claims, wherein the shaft (2) is made of titanium and the attachment (3) is made of steel.

9. The joining method according to one of the preceding claims, **characterized in that** a maximum heating temperature of the attachment (3) is between 50 and 350°C, preferably between 300 and 350°C.

10. The joining method according to one of the preceding claims, wherein the insertion (S2) of the shaft (2) into the opening (4) of the attachment (3) takes place via a linear unit (8).

11. The joining method according to one of claims 1 to 10, wherein the shaft (2) is a handle or handhold and/or the attachment (3) is an operating end or tool head.

12. The joining method according to one of claims 1 to 11, wherein the shaft (2) is made of titanium and/or the attachment (3) is made of steel.

13. Use of a joining method according to one of claims 1 to 12 for manufacturing a medical device (1), in particular a medical instrument or implant, which comprises an attachment (3) and a shaft (2) inserted into an opening (4) of the attachment (3).

## Revendications

1. Procédé d'assemblage destiné à un dispositif médico-technique (1), lequel est un instrument chirurgical ou un implant muni d'une tige (2) et d'un embout (3) fixé sur celle-ci, dans lequel le procédé d'assemblage comprend les étapes suivantes :
le chauffage (S1) de l'embout (3) ou le refroidissement de la tige (2) de sorte qu'un diamètre intérieur (d_{H}) d'une ouverture (4) de l'embout (3) chauffé est supérieur à un diamètre extérieur (D) de la tige (2),
l'insertion (S2) ultérieure de la tige (2) dans l'ouverture (4) de l'embout (3),
le refroidissement (S3) ultérieur de l'embout (3), pour emmancher celui-ci sensiblement sans espace sur la tige (2), ou le réchauffement de la tige (2), pour étendre celle-ci à l'intérieur de l'ouverture (4) de l'embout (3), de sorte que la tige (2) et l'ouverture (4) sont reliées par le biais d'un ajustement pas serrage, **caractérisé en ce que** le procédé d'assemblage comprend en outre l'étape suivante :
la surveillance (S4) de la température de l'embout (3) ou de la tige (2) sur au moins un premier point (P1) à proximité de l'ouverture (4) ou une périphérie extérieure d'une extrémité de la tige (2) à insérer dans l'embout, ainsi qu'un second point sur une zone périphérique de l'embout ou une zone de la tige à distance du premier point (P2, P3).

2. Dispositif d'assemblage selon la revendication 1, comprenant en outre l'étape de surveillance (S4) de la température de l'embout (3) et de préférence d'un temps de chauffage et/ou de refroidissement.

3. Dispositif d'assemblage selon la revendication 2, dans lequel la température de l'embout (3) est surveillé à proximité, de préférence immédiatement, au niveau de l'ouverture (4), ainsi qu'au niveau d'une zone périphérique de l'embout à distance de celle-ci.

4. Dispositif d'assemblage selon l'une quelconque des revendications précédentes, dans lequel pour le chauffage (S1) de l'embout (3) un dispositif de chauffage par induction (5) est utilisé.

5. Dispositif d'assemblage selon l'une quelconque des revendications précédentes, comprenant en outre l'étape de réglage (S5) d'un apport thermique lors du chauffage (S1) en fonction de paramètres de la tige (2) et de l'embout (3).

6. Dispositif d'assemblage selon l'une quelconque des revendications précédentes, dans lequel l'ajustement par serrage est dimensionné de telle manière que la tige (2) et l'embout (3) sont reliés entre eux en fonctionnement axialement et radialement de manière inamovible et de préférence est amovible de manière réversible par un nouveau chauffage.

7. Dispositif d'assemblage selon l'une quelconque des revendications précédentes, dans lequel l'ajustement par serrage présente un serrage compris entre 20 et 55 µm.

8. Dispositif d'assemblage selon l'une quelconque des revendications précédentes, dans lequel la tige (2) est fabriquée en titane et l'embout (3) est fabriqué en acier.

9. Dispositif d'assemblage selon l'une quelconque des revendications précédentes, dans lequel une température de chauffage maximale de l'embout (3) est comprise entre 50 et 350 °C, de préférence entre 300 et 350 °C.

10. Dispositif d'assemblage selon l'une quelconque des revendications précédentes, dans lequel l'insertion (S2) de la tige (2) dans l'ouverture (4) de l'embout (3) s'effectue par le biais d'une unité linéaire (8).

11. Dispositif d'assemblage selon l'une quelconque des revendications 1 à 10, dans lequel la tige (2) est un manche ou une poignée et/ou l'embout (3) est une extrémité de travail ou une tête d'outil.

12. Dispositif d'assemblage selon l'une quelconque des revendications 1 à 11, dans lequel la tige (2) est fabriquée en titane et/ou l'embout (3) est fabriqué en acier.

13. Utilisation d'un procédé d'assemblage selon l'une quelconque des revendications 1 à 12 pour la fabrication d'un dispositif médical (1), en particulier un instrument ou un implant médical, lequel présente un embout (3) et une tige (2) insérée dans une ouverture (4) de l'embout (3).
